(19) Europäisches Patentamt
European Patent Office
Office européen des brevets

(11) EP 0 900 781 B1

(12) **EUROPÄISCHE PATENTSCHRIFT**

(45) Veröffentlichungstag und Bekanntmachung des Hinweises auf die Patenterteilung:
**20.03.2002 Patentblatt 2002/12**

(51) Int Cl.[7]: **C07C 235/34**, C07C 231/02, A61K 31/165, A61K 31/40, A61K 31/48, C09K 15/24, A23B 4/14

(21) Anmeldenummer: **98115422.2**

(22) Anmeldetag: **17.08.1998**

(54) **Phenolsäureamide hydroxysubstituierter Benzylamine**

Phenolic acid amides of hydroxy substituted benzylamines

Amides d'acide phénoliques de benzylamines hydroxy-substituées

(84) Benannte Vertragsstaaten:
**CH DE FR GB IT LI NL**

(30) Priorität: **27.08.1997 DE 19737327**

(43) Veröffentlichungstag der Anmeldung:
**10.03.1999 Patentblatt 1999/10**

(73) Patentinhaber: **HAARMANN & REIMER GMBH**
**D-37601 Holzminden (DE)**

(72) Erfinder: **Ley, Jakob, Dr.**
**37603 Holzminden (DE)**

(74) Vertreter: **Mann, Volker, Dr.**
**Bayer AG**
**Konzernzentrale RP**
**Patente und Lizenzen**
**D-51368 Leverkusen (DE)**

(56) Entgegenhaltungen:
**EP-A- 0 613 879** **NL-A- 9 202 078**

- **NOBUJI NAKATANI ET AL.: "Chemical constituents of peppers (piper spp.) and application to food preservation: Naturally occuring antioxidative compounds" ENVIRONMENTAL HEALTH PERSPECTIVES, Bd. 67, 1986, Seiten 135-142, XP000925839**
- **DATABASE WPI Section Ch, Week 198242 Derwent Publications Ltd., London, GB; Class D13, AN 1982-88998E XP002144864 & JP 57 146563 A (HASEGAWA CO LTD), 10. September 1982 (1982-09-10)**
- **SH Z KOBAYASHI: "Beziehung zwischen chemischer Konstitution und scharfen Geschmack bei Säureamiden." CHEMISCHES ZENTRALBLATT, Bd. 99, Nr. I, 1928, Seiten 1028-1031, XP000925852**
- **UTE MÜHLENBECK ET AL.: "Formation of hydroxycinnamoylamides and ....." PHYTOCHEMISTRY., Bd. 42, Nr. 6, 1996, Seiten 1573-1579, XP000925845 PERGAMON PRESS., GB ISSN: 0031-9422**
- **JOSETTE MARTIN-TANGUY ET AL.: "The distribution of hydroxycinnamic acid amides in flowering plants" PHYTOCHEMISTRY., Bd. 17, Nr. 11, 1978, Seiten 1927-1928, XP000925834 PERGAMON PRESS., GB ISSN: 0031-9422**
- **BURKE T R ET AL: "HYDROXYLATED AROMATIC INHIBITORS OF HIV-1 INTEGRASE" JOURNAL OF MEDICINAL CHEMISTRY,US,AMERICAN CHEMICAL SOCIETY. WASHINGTON, Bd. 38, Nr. 21, 13. Oktober 1995 (1995-10-13), Seiten 4171-4178, XP000569362 ISSN: 0022-2623**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 900 781 B1

## Beschreibung

Die Erfindung betrifft Phenolsäureamide hydroxysubstituierter Benzylamine, ein Verfahren zu deren Herstellung und ihre Verwendung als Antioxidantien oder Radikalfänger, insbesondere in kosmetischen und pharmazeutischen Zubereitungen und Nahrungsmitteln sowie zum Schutz von Zellen und Gewebe von Säugern vor den die Alterung beschleunigenden schädlichen Einflüssen von Radikalen und reaktiven Sauerstoffverbindungen. Die Erfindung betrifft ferner kosmetische und pharmazeutische Zubereitungen umfassend diese Phenolsäureamide.

Die Autoxidation von Lipiden, Proteinen, DNA und anderen Biomolekülen ist in hohem Grade mitverantwortlich für die Alterung physiologischer Systeme, also auch der menschlichen Haut (vgl. z.B. B. Halliwell, M.A. Murcia, S. Chirico, O.I. Aruoma, *Critical Reviews in Food Science and Nutrition*, **1995**, *35 (1&2)*, 7-20 und darin zitierte Literatur). Man vermutet, daß die Schädigung von Biomolekülen bzw. Zellen durch oxidativen Stress zu einer Vielzahl von Krankheiten führt (vgl. M.J. Thomas, *Critical Reviews in Food Science and Nutrition,* **1995**, *35* (1&2), 21-39). Relativ sicher ist dies für einige Krebsarten. Aber auch bei Arthritis und Artherosklerosis vermutet man einen direkten Einfluß.

Die Haut ist als das größte Organ des Menschen und als wichtigste Barriere gegen Umwelteinflüsse besonders betroffen. So werden hier primäre Schäden v.a. durch Strahlung oder Verletzungen hervorgerufen. Der dadurch verursachte Gewebe- oder Zellschaden induziert unter anderem Prozesse, bei denen Radikale erzeugt und/oder Antioxidantien verbraucht oder Promotoren der Autoxidation freigesetzt werden. Dabei können z.B. Eisenionen oder Häm freigesetzt werden, HOCl erzeugende Phagocyten aktiviert werden, die Arachidonsäurekaskade gestartet oder auch die Atmungskette unterbrochen werden, wobei dann vermehrt reaktive Sauerstoffspezies oder Radikale freigesetzt werden. Durch diese werden Lipide der Zellmembranen, Proteine (u.a. das intrazellulare Fibrin, Enzyme und das interzellulare Stützprotein Kollagen), Polysaccharide (z.B. die gelbildende Hyaluronsäure) und auch die DNA in den Dermiszellen geschädigt. Wenn diese Schäden durch endogene Prozesse nicht mehr ausreichend ausgeglichen werden können, altert die Haut vorzeitig. Dies zeigt sich v.a. in Form einer Erschlaffung und damit einer Faltenbildung. Zusätzlich können Zellen außer Kontrolle geraten und Tumoren wie z.B. die bösartigen Melanome bilden.

Die bekannteste Autoxidation ist die von Lipiden, insbesondere von ungesättigten Fettsäuren, wodurch vor allem die Membranen von sonst intakten Zellen geschädigt werden. Die Autoxidation läuft über einen Radikalkettenmechanismus ab, den man in die drei Schritte Initiierung, Propagierung (Kettenfortpflanzung) und Abbruchreaktion unterteilen kann (s. Lehrbücher der organischen Chemie). Die Radikalinitiierung und die Kettenfortpflanzung wird durch Schwermetallionen, insbesondere die in physiologischen Systemen vorkommenden Eisen- und Kupferionen, stark promoviert.

Die Oxidation von Fetten oder anderen Biomolekülen spielt aber auch im Produktschutz z.B. von Kosmetika, Pharmazeutika oder Nahrungsmitteln eine große Rolle. Prinzipiell finden hier ähnliche Reaktionen wie oben beschrieben statt, wobei die Radikalbildung insbesondere durch Erwärmung, Schwermetallionen oder UV-Licht initiiert wird.

Daher ist es erstrebenswert, Substanzen zu finden, die in physiologischen Systemen die natürlichen Abwehrmechanismen gegen freie Radikale und reaktive Sauerstoffverbindungen unterstützen oder als Schutzstoffe in Kosmetika, Pharmazeutika oder Nahrungsmitteln deren oxidationsempfindlichen Bestandteile vor Autoxidation schützen.

Antioxidantien sind definiert als Substanzen, die in im Vergleich zu dem oxidierbaren Substrat kleinen Konzentrationen die Oxidation signifikant verzögern oder gänzlich verhindern. Viele Antioxidantien fungieren gleichzeitig als Radikalfänger und/oder als Komplexbildner für Schwermetallionen.

Einige natürliche und sehr wichtige Antioxidantien oder Radikalfänger sind die Tocopherole (Vitamin E), L-Ascorbinsäure (Vitamin C) und Glutathion. Weiterhin spielen Ubichinol, β-Carotin und Bilirubin (Abbauprodukt von Porphyrin-Derivaten) als Antioxidantien *in vivo* eine Rolle (vgl. C.-M. Andersson, A. Hallberg, T. Högberg, "Advances in the Development of Pharmaceutical Antioxidants", in *Adv. Drug Res.,* B. Testa, U.A. Meyer (Hrsg.), Academic Press, London, **1996**, S. 65-180). Daneben sind noch die mehrwertigen Säuren wie z.B. Zitronensäure und Aminosäuren zu erwähnen, die chelatisierend wirken und damit Metallionen maskieren können. Ein weiteres Antioxidans speziell in der Haut ist das in den Melanocyten gebildete Melanin, ein meist braun-schwarzes Polymer, daß durch Oxidation und Polymerisation aus aromatischen Aminosäuren wie L-Tyrosin gebildet wird (vgl. M.R. Chedekel, *Cosmetics & Toiletries* **1996**, *111*(1), 71-74).

Die wichtigsten nichtnatürlichen Antioxidantien, die vor allem in der Nahrungsmittelindustrie zur Stabilisierung von Fetten und Ölen eingesetzt werden, sind 2- bzw. 3-*tert*-Butyl-4-methoxyphenol (1:9-Gemisch, butyliertes Hydroxyanisol, BHA) und 2,6-Di-*tert*-butyl-4-methylphenol (butyliertes Hydroxytoluol, BHT), Gallussäurepropylester (PG), Gallussäuredodecanylester (DG) und 2-*tert*-Butyl-1,4-dihydroxybenzol (TBHQ). Viele der primär gebildeten Produkte aus Radikalrekombinationen der Phenoxylradikale sind selber wieder Antioxidantien. Unter anderem aus diesem Grund werden die Antioxidantien häufig als Gemische eingesetzt, da sie gegenseitige synergistische Wirkung zeigen. Einige dieser synthetischen Antioxidantien sind allerdings aus toxikologischer Sicht als unsicher eingestuft worden (vgl. S.M. Barlow, "Toxicological Aspects of Antioxidants Used as Food Additives", in *Food Antioxidants*, B.J.F. Hudson (Hrsg.), Elsevier, London, **1990**, S. 253-307).

Zu den natürlich v.a. in Pflanzen vorkommenden Antioxidantien gehören z.B. auch die ω-Phenylalkylsäurederivate wie p-Cumarsäure, Kaffeesäure, Ferulasäure, Sinapinsäure und Analoge sowie einige Flavonoide.

In der Natur findet man einige Ferulasäureamide von hydroxysubstituierten 2-(Phenyl-)-ethylaminen (Martin-Tanguy, J.; Cabanne, F.; Pedrizet, E.; Martin, C., *Phytochemistry* **1978**, *17* (11), 1927-1928); von diesen Verbindungen wurde v.a. 4-Hydroxy-3-methoxy-E-zimtsäure-2-(4-hydroxyphenyl)ethylamid in einer Vielzahl von Pflanzen gefunden, so z.B. in der Tomate. Die Verbindung wurde auch aus dem schwarzen Pfeffer isoliert und die antioxidative Wirkung beschrieben (JP 57,146,563; Nakatani, N.; Inatani, R.; Ohta, H.; Nishioka, A., *EHP, Envir. Health Perspect.* **1986**, *67*, 135-142). Allerdings ist die Verbindung nur in kleinen Mengen in den Pflanzen zu finden (0,03 %) und nicht ausreichend verfügbar. Zudem wirkt sie, wie wir feststellen konnten, nicht besser als $\alpha$-Tocopherol (vgl. Experimente 1 bzw. 2).

Einige weitere Ferulasäureamide und Kaffeesäureamide von Phenethylaminen wurden aus verschiedenen Pflanzen isoliert; so z.B. 3,4-Dihydroxy-E-zimtsäure-2-(4-hydroxyphenyl)ethylamid aus der Roßkastanie (Martin-Tanguy, J.; Cabanne, F.; Pedrizet, E.; Martin, C., *Phytochemistry* **1978**, *17* (11), 1927-1928) oder aus *Annona crassiflora* Mart. (Santos, L.P.; Boaventura, M.A.D.; de Oliveira, A.B.; Cassady, J.M., *Planta Medica* **1996**, *62* (1), 76-77). Bei einigen Pflanzen, v.a. bei Nachtschattengewächsen, werden sie als Antwort auf eine Verletzung gebildet (Beispiel: 4-Hydroxy-3-methoxy-E-zimtsäure-2-(4-hydroxyphenyl)-2-hydroxyethylamid in der Kartoffel: Negrel, J.; Pollet, B.; Lapierre, C., *Phytochemistry* **1996**, *43* (6), 1195-1199).

Die Wirkung von einigen u.a. im Hafer vorkommenden N-Hydroxycinnamoylhydroxyanthranilsäuren als Insektizide wird z.B. in NL 92,02,078 beschrieben.

Einige mehrfach hydroxylierte Dihydrozimtsäureamide von 2-(Phenyl)ethylaminen sind in der Literatur beschrieben, so z.B. 3-(3,4-Dihydroxyphenyl)propansäure-2-(3,4-dihydroxyphenyl)ethylamid als HIV-Integraseinhibitor (Burke, T.R.; Fresen, M.R.; Mazumder, A; Wang, J.; Carothers, A.M.; Grunberger, D.; Driscol, J.; Kohn, K.; Pommier, Y., *J Med Chem.,* **1995**, *38* (21), 4171-4178).

Als antiallergische Wirkstoffe wurden 4-Hydroxy-3-methoxyphenylessigsäure-(4-hydroxy-3-methoxybenzyl) amid bzw. dessen N-Methylderivat beschrieben (WO 92 20,645).

Zumindest die in der Natur vorkommenden, oben genannten Phenolsäureamide, sind bisher nur in kleinen Mengen isoliert bzw. dargestellt worden und sind damit nur unzureichend verfügbar.

In der Literatur ist bisher noch kein allgemein anwendbares und befriedigendes Verfahren zur Synthese der oben genannten Verbindungen beschrieben worden. Die klassische Kupplung der Säurechloride mit Aminen scheitert häufig an Nebenreaktionen, insbesondere Selbstkondensation der Phenolsäuren, die auch durch Anwendung von O-Acetylschutzgruppen nicht vollständig verdrängt werden kann und geringe Ausbeuten liefert (<35 %, vgl. Tseng, C. F.; Iwakami, S.; Mikajiri, A.; Shibuya, M.; Hanaoka, F.; Ebizuka, Y.; Padmawinata, K.; Sankawa, U., *Chem. Pharm. Bull.* **1992**, *40,* 396-400).

Eine Synthese von einigen wenigen Ferula- und Kaffeesäuretyramiden durch Aminolyse der N-Hydroxysuccinimidylester für analytische Zwecke wurde in der Literatur beschrieben (vgl. Mühlenbeck, U.; Kortenbusch, A.; Barz, W., *Phytochemistry* **1996**, *42*,1573-1579). Es erfolgten jedoch keine Ausbeuteangaben.

Die Aufgabe der vorliegenden Erfindung liegt darin, aufbauend auf den obigen Erkenntnissen neue Antioxidantien mit starker spezifischer radikalfangender und anti-oxidativer Wirkung zu entwickeln, wobei die Moleküle eine Molekularmasse unter 400 Da besitzen und durch synthetische Methoden leicht zugänglich sein sollen.

Die Erfindung betrifft Phenolsäureamide hydroxysubstituierter Benzylamine der allgemeinen Formel I

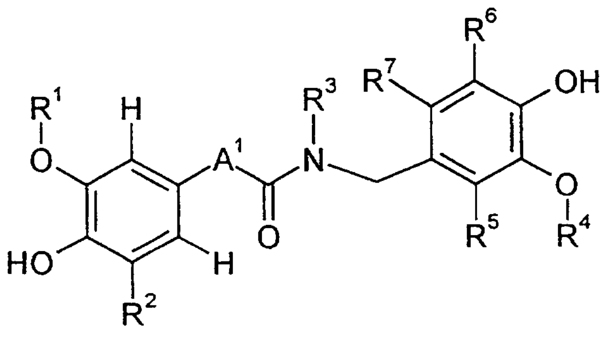

(I)

wobei

$A^1$ eine Gruppe $-CH_2-$, eine Gruppe $-CH_2-CH_2-$ oder eine Gruppe $-CH=CH-$ darstellt, wobei die letztere sowohl in der (Z)- als auch in der (E)-Konfiguration vorliegen kann,

und

$R^1$ ein Wasserstoffatom oder eine Alkylrest mit 1 bis 4 Kohlenstoffatomen darstellt,

und

$R^2$ ein Wasserstoffatom oder eine Gruppe -O-$R^8$ darstellt, in der $R^8$ ein Wasserstoffatom oder ein Alkylrest mit 1 bis 4 Kohlenstoffatomen bedeutet,

und

$R^3$ ein Wasserstoffatom, einen Acyl-, Alkyl- oder Alkenylreste bis 22 Kohlenstoffatome oder eine Gruppe $-(CH_2-CH_2-O-)_nH$ darstellt, wobei n 1 bis 15 sein kann,

und

$R^4$ ein Wasserstoffatom oder eine Alkylrest mit 1 bis 4 Kohlenstoffatomen darstellt, mit der Maßgabe, daß mindestens einer der beiden Reste $R^1$ und $R^4$ ein Wasserstoffatom darstellt,

und

$R^5$, $R^6$ und $R^7$ unabhängig voneinander Wasserstoffatome oder Gruppen -O-$R^9$ darstellen, wobei $R^9$ ein Wasserstoffatom oder ein Alkylrest mit 1 bis 4 Kohlenstoffatomen ist.

Bevorzugt sind Verbindungen der allgemeinen Formel I, wobei

$A^1$ eine Gruppe $-CH_2-$, eine Gruppe $-CH_2-CH_2-$ oder eine Gruppe -CH=CH- darstellt, die in der (E)-Konfiguration vorliegt,

und

$R^1$ ein Wasserstoffatom oder eine Methylgruppe darstellt,

und

$R^2$ ein Wasserstoffatom, eine Hydroxygruppe oder eine Gruppe $-O-CH_3$ darstellt,

und

$R^3$ ein Wasserstoffatom, Alkyl- oder Alkenylreste bis 22 Kohlenstoffatome oder eine Gruppe $-(CH_2-CH_2-O-)_nH$ darstellt, wobei n von 1 bis 15 sein kann,

und

$R^4$ ein Wasserstoffatom oder eine Methylgruppe darstellt, mit der Maßgabe, daß mindestens einer der beiden Reste $R^1$ und $R^4$ ein Wasserstoffatom darstellt,

und

$R^5$, $R^6$ und $R^7$ unabhängig voneinander Wasserstoffatome, Hydroxygruppen oder Gruppen $-O-CH_3$ darstellen.

Die insbesondere bevorzugten Verbindungen der allgemeinen Formel (I) umfassen z.B.:

4-Hydroxy-3 -methoxy-*E*-zimtsäure-(3,4-dihydroxybenzyl)-amid,
3,5-Dimethoxy-4-hydroxy-*E*-zimtsäure-(3,4-dihydroxybenzyl)-amid,
4-Hydroxy-3-methoxy-*E*-zimtsäure-(2,3,4-trihydroxybenzyl)-amid,
3,5-Dimethoxy-4-hydroxy-*E*-zimtsäure-(2,3,4-trihydroxybenzyl)-amid,
4-Hydroxy-3-methoxy-*E*-zimtsäure-(3,4,5-trihydroxybenzyl)-amid,
4-Hydroxy-3-methoxyphenylessigsäure-(3,4-dihydroxybenzyl)-amid,

3-(4-Hydroxy-3-methoxyphenyl)-propansäure-(3,4-dihydroxybenzyl)-amid,
3 -(3,4-Dihydroxyphenyl)-propansäure-(4-hydroxy-3-methoxybenzyl)-amid,
3-(3,4-Dihydroxyphenyl)-propansäure-(3,4-dihydroxybenzyl)-amid,
3-(3,4-Dihydroxyphenyl)-propansäure-(2,3,4-trihydroxybenzyl)-amid,
3,4-Dihydroxy-*E*-zimtsäure-(3,4-dihydroxybenzyl)-amid,
3,4-Dihydroxy-*E*-zimtsäure-(4-hydroxy-3-methoxybenzyl)-amid,
3,4-Dihydroxy-*E*-zimtsäure-(3,5-dimethoxy-4-hydroxybenzyl)-amid,
3,4-Dihydroxy-*E*-zimtsäure-(2,3,4-trihydroxybenzyl)-amid,
3,4-Dihydroxyphenylessigsäure-(4-hydroxy-3-methoxybenzyl)-amid,
3,4-Dihydroxyphenylessigsäure-(3,4-dihydroxybenzyl)-amid,

sind jedoch nicht darauf beschränkt.

Überraschenderweise wurde nun gefunden, daß die erfindungsgemäßen Phenolsäureamide hydroxysubstituierter Benzylamine besonders starke Radikalfänger und starke Antioxidantien sind. Insbesondere sind sie wesentlich bessere Antioxidantien und Radikalfänger als die natürlich vorkommenden Hydroxyzimtsäureamide von 2-(Phenyl) ethylaminen (s.o.) und die meisten üblichen Antioxidantien (s. Experimente).

Besonders vorteilhaft als Antioxidantien oder Radikalfänger sind die Verbindungen im Sinne der Erfindung mit mehr als drei Hydroxygruppen.

Die erfindungsgemäßen Phenolsäureamide hydroxysubstituierter Benzylamine der allgemeinen Formel I können mit den an sich bekannten üblichen Amidsyntheseverfahren hergestellt werden, dergestalt, daß man eine aktivierte, gegebenenfalls an den phenolischen OH-Gruppen geschützte Phenolsäure mit einem gegebenenfalls an den phenolischen OH-Gruppen geschützen Benzylamin oder dessen Ammoniumsalz, gegebenenfalls in Gegenwart von Lösemitteln und Hilfsbasen umsetzt. Als aktivierte Säurederivate können die Säurechloride, die Säureanhydride oder Säureester von z.B. gegebenenfalls substituierten Phenolen, N-Hydroxysuccinimid oder N-Hydroxybenzotriazol verwendet werden. Als Schutzgruppen verwendet man bevorzugt Acyl-, Carbamat- oder Ethergruppen, z.B. Acetyl-, Benzoyl-, Methoxycarbonyl-, tert.-Butoxycarbonyl-, Allyl- oder Benzylgruppen. Als Lösungsmittel können z.B. Wasser, Aceton, Dioxan, Dimethylformamid, Tetrahydrofuran, Essigsäureethylester, Chloroform oder auch Gemische der letztgenannten Lösemittel verwendet werden. Als Hilfsbasen können z.B. Ammonium-, Alkalimetall- oder Erdalkalimetallcarbonate, -hydrogencarbonate, -hydroxide und tert. Amine verwendet werden.

Die erfindungsgemäßen Phenolsäureamide hydroxysubstituierter Benzylamine der allgemeinen Formel I werden besonders bevorzugt aus gegebenenfalls an den Hydroxygruppen mit Acetyl- oder Methoxycarbonylgruppen blokkierten Phenolsäure-N-succinimidylestern mit einem hydroxysubstituierten Benzylamin oder dessen Ammoniumsalz in einem wasserhaltigen Lösungsmittelgemisch, bevorzugt einem Wasser/1,4-Dioxan-Gemisch mit einer der oben genannten Hilfsbasen bei 5 bis 100°C hergestellt.

Als aktivierte Phenolsäure-N-succinimidylester werden insbesondere 4-Hydroxy-3-methoxy-E-zimtsäure-N-succinimidylester, 3,5-Dimethoxy-4-hydroxy-E-zimtsäure-N-succinimidyl-ester, 3,4-Bis(acetyloxy)-E-zimtsäure-N-succinimidyl-ester, 3,4-Bis(methoxycarbonyloxy)-E-zimtsäure-N-succinimidylester, 4-Hydroxy-3-methoxyphenylessigsäure-N-succinimidylester, 3,4-Bis(methoxycarbonyloxy)-phenylessigsäure-N-succinimidylester, 3-(4-Hydroxy-3-methoxyphenyl)-propansäure-N-succinimidylester und 3-(3,4-Dihydroxyphenyl)-propansäure-N-succinimidylester vewendet.

Als hydroxysubstituierte Benzylamine werden insbesondere 3,4-Dihydroxybenzylamin, 4-Hydroxy-3-methoxybenzylamin, 3,4-Dimethoxy-4-hydroxybenzylamin, 2,3,4-Trihydroxybenzylamin, oder 3,4,5-Trihydroxybenzylamin oder die jeweiligen Ammoniumsalze verwendet.

Die erfindungsgemäßen Phenolsäureamide können aber auch durch direkte Kondensation der freien Säuren mit den freien Aminen mit oder ohne Lösemittel erhalten werden. Als Kondensationsmittel können z.B. Carbodiimide, bevorzugt N,N'-Dicyclohexylcarbodiimid, und als Lösemittel z.B. 1,4-Dioxan, Tetrahydrofuran, tert.-Butylmethylether oder Essigsäureethylester verwendet werden.

Die erfindungsgemäßen Phenolsäureamide erhält man aus diesen Reaktionsgemischen durch dem Fachmann wohlvertraute Reinigungsschritte; gegebenenfalls müssen noch vorhandene Schutzgruppen mit an sich bekannten Methoden abgespalten werden.

Die erfindungsgemäßen Phenolsäureamide hydroxysubstituierter Benzylamine der allgemeinen Formel I können als Antioxidantien oder Radikalfänger zum Schutz gegen Oxidation und Photooxidation verwendet werden. Bevorzugt können sie in kosmetischen, pharmazeutischen oder dermatologischen Formulierungen oder in Nahrungsmitteln eingesetzt werden. Besonders bevorzugt werden die erfindungsgemäßen Phenolsäureamide in kosmetischen und dermatologischen Formulierungen verwendet, die wie üblich zusammengesetzt sind und zur Behandlung, der Pflege und der Reinigung der Haut und/oder der Haare und als Schminkprodukte in der dekorativen Kosmetik dienen.

Dementsprechend betrifft die vorliegende Erfindung auch kosmetische und pharmazeutische Zusammensetzungen, insbesondere kosmetische und dermatologische Zusammensetzungen, welche die erfindungsgemäßen Phenolsäureamide in einer wirksamen Menge, nebst anderen, ansonsten üblichen Zusammensetzungsbestandteilen, umfassen. Sie enthalten 0,0001 Gew.-% bis 30 Gew.-%, bevorzugt 0,0001 bis 20 Gew.-%, insbesondere aber 0,0001 Gew.-% bis 5 Gew.-%, bezogen auf das Gesamtgewicht der Formulierung, an den erfindungsgemäßen Phenolsäureamiden der allgemeinen Formel I und können dabei sowohl als "Wasser in Öl" als auch "Öl in Wasser"-Emulsionen vorliegen. Weitere übliche kosmetische Hilfs- und Zusatzstoffe können in Mengen von 5 - 95 Gew.-%, vorzugsweise 10 - 80 Gew.-%, bezogen auf das Gesamtgewicht der Formulierung, enthalten sein. Ferner können die Formulierungen Wasser in einer Menge bis zu 99 Gew.-%, vorzugsweise 5- 80 Gew.-%, bezogen auf das Gesamtgewicht der Formulierung, aufweisen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfs- und Zusatzstoffe enthalten, wie sie üblicherweise in solchen Zubereitungen vewendet werden, z.B. Konservierungsmittel, Bakterizide, Fungizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Bevorzugt können die erfindungsgemäßen Phenolsäureamide hydroxysubstituierter Benzylamine sowohl untereinander als auch mit anderen Antioxidantien kombiniert werden. Insbesondere können die erfindungsgemäßen Phenolsäureamide hydroxysubstituierter Benzylamine untereinander als auch mit Tocopherolen (Vitamin E), Tocopherolderivaten, Tocotrienolen, Ascorbinsäure (Vitamin C), Ascorbinsäurederivaten, Carotinoiden, Vitamin A oder dessen Derivaten, BHT, BHA, Gallussäureestern, Flavonoiden wie z.B. Quercetin oder Myricetin, Catechinen wie z.B. Epicatechin, Epicatechingallat, Epigallocatechin oder Epigallocatechingallat, Thiolen wie z.B. Glutathion oder anderen üblichen Antioxidantien kombiniert werden.

Die Menge der vorgenannten beispielhaften Antioxidantien (eine oder meherere Verbindungen), welche nicht mit erfindungsgemäßen Phenolsäureamiden hydroxysubstituierter Benzylamine identisch sind, kann in den erfindungsgemäßen Zubereitungen 0,0001 bis 30 Gew.-%, bevorzugt 0,0001 bis 20 Gew-%, besonders bevorzugt 0,0001 bis 5 Gew.-%, bezogen auf das Gesamtgewicht der Zubereitung, betragen.

Die erfindungsgemäßen Phenolsäureamide hydroxysubstituierter Benzylamine können aber auch zusammen mit UVA- und/oder UVB-Filtersubstanzen in den erfindungsgemäßen kosmetischen oder dermatologischen Formulierungen eingesetzt werden, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man Sonnenschutzmitteln für Haut und Haar erhält.

In einer bevorzugten Ausführungsform können die Phenolsäureamide hydroxysubstituierter Benzylamine im Sinne der Erfindung schließlich auch in Squalen oder Squalan gelöst und gegebenfalls mit den anderen Inhaltsstoffen zusammen mit flüchtigen oder nichtflüchtigen Siliconverbindungen als wasserfreie oder fast wasserfreie Systeme formuliert werden.

Als vorteilhafte Verkörperung der vorliegenden Erfindung wird die Verwendung erfindungsgemäßer Phenolsäureamide hydroxysubstituierter Benzylamine zum Schutz von Geweben und Zellen von Säugern, insbesondere der Haut und/oder der Haare, vor oxidativer Beanspruchung und dem schädlichen Einfluß von Radikalen angesehen.

Ebenso umfaßt die vorliegende Erfindung auch ein Verfahren zum Schutze kosmetischer oder dermatologischer Zubereitungen gegen Oxidation oder Photooxidation, wobei es sich bei diesen Zubereitungen z.B. um Zubereitungen zur Behandlung und Pflege der Haut oder der Haare oder ferner auch Schminkprodukte handeln kann, deren Bestandteile Stabilitätsprobleme aufgrund von Oxidation bzw. Photooxidation bei der Lagerung mit sich bringen, dadurch gekennzeichnet, daß die kosmetischen oder dermatologischen Zubereitungen einen wirksamen Gehalt an erfindungsgemäßen Phenolsäureamide hydroxysubstituierter Benzylamine aufweisen.

Die erfindungsgemäßen Phenolsäureamide hydroxysubstituierter Benzylamine können somit auch verwendet werden zur Herstellung pharmazeutischer, insbesondere dermatologischer Zusammensetzungen zum Schutz von Zellen und Geweben von Säugern, insbesondere des Menschen, gegenüber dem schädlichen Einfluß von freien Radikalen und reaktiven Sauerstoffspezies.

Die Menge an erfindungsgemäßen Phenolsäureamiden hydroxysubstituierter Benzylamine in diesen Zubereitungen beträgt 0,0001 Gew.-% bis 30 Gew.-%, bevorzugt 0,0001 bis 20 Gew.-%, besonders bevorzugt 0,0001 Gew.-% bis 5 Gew.-%, bezogen auf das Gesamtgewicht der Zubereitungen.

Die vorliegende Erfindung umfaßt auch die Verwendung der erfindungsgemäßen Phenolsäureamide zum Schutz von Nahrungsmitteln, bevorzugt Fette oder Fettderivate wie zum Beispiel Fettsäuren oder Fettalkohole enthaltenden Nahrungsmitteln, insbesondere aber Nahrungsmitteln, die Fette oder Fettderivate mit oxidierbaren Doppelbindungen enthalten, vor oxidativer Beanspruchung und vor dem schädigenden Einfluß von Radikalen.

Vorzugsweise beträgt die Menge an erfindungsgemäßen Phenolsäureamiden in Nahrungsmitteln 0,0001

Gew.-% bis 30 Gew.-%, besonders bevorzugt 0,0001 bis 20 Gew.-%, insbesondere aber 0,0001 Gew.-% bis 5 Gew.-%, bezogen auf das Gesamtgewicht des Nahrungsmittels.

Bevorzugt können die erfindungsgemäßen Phenolsäureamide für die erfindungsgemäße Verwendung zum Schutz von Nahrungsmitteln untereinander als auch mit anderen Antioxidantien, wie oben beispielhaft aufgeführt, kombiniert werden.

Die Menge der obengenannten beispielhaften Antioxidantien (eine oder mehrere Verbindungen), welche nicht mit erfindungsgemäßen Phenolsäureamiden identisch sind, beträgt in den Nahrungsmitteln vorzugsweise 0,0001 bis 30 Gew.-%, besonders bevorzugt 0,0001 bis 20 Gew-%, insbesondere 0,0001 bis 5 Gew.-%, bezogen auf das Gesamtgewicht der Nahrungsmittel.

## Beispiele

Die nachfolgenden Beispiele sollen die vorliegende Erfindung verdeutlichen, ohne sie einzuschränken.

### 4-Hydroxy-3-methoxy-*E*-zimtsäure-(3,4-dihydroxybenzyl)-amid (1)

Ferulasäure-*N*-succinimidylester (503 mg, 1,73 mmol) und 3,4-Dihydroxybenzylaminhydrobromid (402 mg, 1,83 mmol) wurden unter Stickstoff in Wasser (10 ml) und 1,4-Dioxan (10 ml) gelöst. Nach Zugabe von $NaHCO_3$ (160 mg, 1,90 mmol) wurde die Lösung 2,5 - 3 h bei ca. 70 °C gerührt und abkühlen gelassen. Die Mischung wurde mit Essigsäureethylester extrahiert, die vereinigten organischen Phasen mit Salzsäure (10 %,), Wasser und gesättigter wäßriger NaCl-Lsg. gewaschen, über $Na_2SO_4$ getrocknet, abfiltriert, das Filtrat im Vakuum eingedampft und der Rückstand chromatographisch an Kieselgel gereinigt. Ausbeute: 540 mg (99 %); $^1$H-NMR (400 MHz, $CDCl_3$): δ = 7,55 (1H, d, 15,5 Hz), 7,02 (1H, dd, 8,2 Hz, 1,9 Hz), 6,93 (1H, s, 1,9 Hz), 6,89 (1H, d, 8,3 Hz), 6,88 (1H, d, 2Hz), 6,82 (1H, d, 8,1 Hz), 8,72 (1H, dd, 8,1 Hz, 2,0 Hz), 6,51 (1H, s), 6,21 (1H, d, 15,5 Hz), 5,82 (1H, t, 5,8 Hz) 5,79 (1H, s), 5,45 (1H, s), 4,45 (2H, d, 5,8 Hz), 3,90 (3H, s) ppm; $^{13}$C-NMR (100 MHz, $CDCl_3$): δ = 165,1, 148,2, 147,8, 145,1, 144,1, 139,0, 130,3, 126,4, 121,4, 119,0, 118,3, 115,6, 115,3, 115,0, 110,8, 55,5, 41,9 ppm; MS (EI): *m*/*z* = 315,1 (100 %), 178,1 (66 %), 177,1 (84 %), 150,1 (36 %), 147,0 (22 %), 145,1 (46 %), 138,1 (83 %), 117,1 (24 %), 89,0 (24 %), 77,1 (22 %).

In analoger Weise wurden die folgenden Verbindungen in Form von farblosen oder schwach gelblichen Kristallen oder amorphen Festkörpern erhalten:

### 3,5-Dimethoxy-4-hydroxy-*E*-zimtsäure-(3,4-dihydroxybenzyl)-amid (2):

$^1$H-NMR (400 MHz, $CD_3OD$): δ = 7,46 (1H, d, 15,6 Hz), 6,85 (2H, s), 6,75 (1H, d, 2,1 Hz), 6,72 (1H, d, 8,1 Hz), 6,64 (1H, dd, 8,1 Hz, 2,0 Hz), 6,47 (1H, d, 15,6 Hz), 4,33 (2H, s), 3,86 (6H, s) ppm; $^{13}$C-NMR (100 MHz, $CD_3OD$): δ = 168,8 (s), 149,5 (d), 146,5 (s), 145,7 (s), 142,5 (2*s), 139,0 (s), 131,4 (s), 127,3 (s), 120,3 (d), 119,2 (d), 116,3 (d), 116,0 (d), 106,5 (2*d), 56,8 (2*q), 44,1 (t) ppm; MS (EI): *m*/*z* = 345,1 (43 %), 209,2 (13 %), 208,1 (100 %), 207,1 (20 %), 180,1 (43 %), 177,1 (21 %), 175,1 (13 %), 165,1 (16 %), 138,1 (37 %), 123,1 (16 %).

### 4-Hydroxy-3-methoxy-*E*-zimtsäure-(2,3,4-trihydroxybenzyl)amid (3):

$^1$H-NMR(400 MHz, $CD_3OD$): δ = 7.47 (1H, d, 14,5 Hz), 7,11 (1H, d, ≈ 2 Hz), 7,03 (1H, dd, 8 Hz, ≈ 2 Hz), 6,77 (1H, d, 8 Hz), 6,53 (1H, d, 8 Hz), 6,41 (1H, d, 14,5 Hz), 6,34 (1H, d, 8 Hz), 4,33 (2H, s), 3,86 (3H, s) ppm; MS (ESI pos.) *m*/*z* = 332,1 (100 %, $[M+H]^+$), 333,0 (20 %, $[M+2H]^+$).

### 3,5-Dimethoxy-4-hydroxy-*E*-zimtsäure-(2,3,4-trihydroxybenzyl)amid (4):

$^1$H-NMR (400 MHz, $CD_3OD$): δ = 7,46 (1H, d, 14 Hz), 6,85 (2H, s), 6,55 (1H, d, 9 Hz), 6,45 (1H, d, 14 Hz), 6,32 (1H, d, 9 Hz), 4,33 (2 H, s), 3,87 (6H, s); MS (ESI pos.) *m*/*z* = 362,0 (100 %, $[M+H]^+$), 362,9 (20 %, $[M+2H]^+$).

### 4-Hydroxy-3-methoxy-*E*-zimtsäure-(3,4,5-trihydroxybenzyl)amid (5):

$^1$H-NMR (400 Mhz, $CDCl_3$): δ = 7,46 (1H, d, 15 Hz), 7,12 (1H, d, 2Hz), 7,03 (1H, dd, 8,5 Hz, 2 Hz), 6,78 (1H, d, 8,5 Hz), 6,45 (1H, d, 15 Hz), 6, 32 (2H, s), 4,26 (2H, s) 3,88 (3H, s) ppm; MS (APCI-) *m*/*z* = 660,8 (100 %, $[2M+H]^-$), 330,1 (99,9 % $[M-H]^-$).

### 4-Hydroxy-3-methoxyphenylessigsäure-(3,4-dihydroxybenzyl)-amid (6):

$^1$H-NMR (400 MHz, $CDCl_3$): δ = 6,84 (1H, d, 1,7 Hz), 6,72 (1H, d, 7,9 Hz), ca. 6,70 (2H, m), 6,68 (1H, d, 8,0

Hz), 6,56 (1H, dd, 8,0 Hz, 2,1 Hz), 4,20 (2H, s), 3,80 (3H, s), 3,42 (2H, s) ppm; $^{13}$C-NMR (100 MHz, $CDCl_3$): δ = 174,3 (s), 149,0 (s), 146,6 (s), 131,4 (s), 128,3 (s), 122,8 (d), 120,2 (d), 116,2 (2*d), 116,1 (d), 113,6 (d), 56,4 (q), 44,1 (t), 43,5 (t) ppm; MS (EI): *m/z* = 303 (41 %), 138 (48 %), 137 (100 %), 123 (76 %), 122 (14 %), 94 (10 %), 77 (13 %).

**3-(4-Hydroxy-3-methoxyphenyl)-propansäure-(3,4-dihydroxybenzyl)-amid (7):**

$^1$H-NMR (400 MHz, $CD_3OD$): δ = 6,76 (1H, d, 2,0 Hz), 6,69 - 6,61 (4H, m), 6,45 (1H, dd, 2,1 Hz, 8,0 Hz), 4,16 (2H, s), 3,78 (3H, s), 2,84 (2H, m), 2,46 (2H, m) ppm; $^{13}$C-NMR (100 MHz, $CD_3OD$): δ = 175,1 (s), 148,9 (s), 146,4 (s), 145,9 (s), 145,6 (s), 133,7 (s), 131,3 (s), 121,9 (d), 120,1 (d); 116,3 (d), 117,0 (d), 116,0 (d), 113,2 (d), 56,4 (q), 43,9 (t), 39,4 (t), 32,6 (t) ppm; MS (APCI +): *m/z* = 318,0 (100 %, $[M+H]^+$), 634,7 (40 %, $[2M+H]^+$).

**3-(3,4-Dihydroxyphenyl)-propansäure-(4-hydroxy-3-methoxybenzyl)-amid (8):**

$^1$H-NMR (400 MHz, $CD_3OD$): δ = 6,76 (1H, d, 2,0 Hz), 6,70 (1H, d, 8,1 Hz), 6,64 (1H, d, 8,1 Hz), 6,64 (1H, d, 2,1 Hz), 6,58 (1H, dd, 81, Hz, 2,0 Hz), 6,50 (1H, dd, 8,1 Hz, 2,1 Hz), 4,22 (2H, s), 3,79 (3H, s), 2,77 (2H, m), 2,45 (2H, m) ppm; $^{13}$C-NMR (100 MHz, $CD_3OD$): δ = 175,2 (s), 149,0 (s), 146,8 (s), 146,3 (s), 144,7 (s), 133,7 (s), 131,3 (s), 121,4 (d), 120,6 (d), 116,6 (d), 116,3 (d), 116,1 (d), 112,4 (d), 56,4 (q), 44,1 (t), 39,3 (t), 32,4 (t) ppm; MS (APCI +): *m/z* = 317,9 (100 %, $[M+H]^+$), 635,0 (45 %, $[2M+H]^+$).

**3-(3,4-Dihydroxyphenyl)-propansäure-(3,4-dihydroxybenzyl)-amid (9):**

$^1$H-NMR (400 MHz, $CD_3OD$): δ = 6,68 (1H, d, 8,1 Hz), 6,67 (1H, d, 2,0 Hz), 6,65 (1H, d, 8,1 Hz), 6,64 (1H, d, 2,1 Hz), 6,51 (1H, dd, 8,1 Hz, 2,1 Hz), 6,48 (1H dd, 8,1 Hz, 2,1 Hz), 4,16 (2H, s), 2,77 (2H, m), 2,43 (2H m) ppm; $^{13}$C-NMR (100 MHz, $CD_3OD$): δ = 175,2 (s), 146,3 (s), 146,2 (s), 145,6 (d), 144,6 (s), 133,8 (s), 131,4 (s), 120,7 (d), 120,2 (d), 116,6 (d), 116,4 (d), 116,3 (d), 116,0 (d), 43,9 (t), 39,5 (t), 32,5 (t) ppm; MS (ESI -): *m/z* = 302,3 (100 %, $[M-H]^-$), 605,0 (35 %, $[2M-H]^-$).

**3-(3,4-Dihydroxyphenyl)-propansäure-(2,3,4-trihydroxybenzyl)-amid (10):**

$^1$H-NMR (400 MHz, $CD_3OD$): δ = 6,61 (1H, d, 2,1 Hz), 6,61 (1H, d, 8 Hz), 6,47 (1H, dd, 8,0 Hz, 2,1 Hz), 6,41 (1H, d, 8,3 Hz), 6,28 (1H, d, 8,3 Hz), 4,18 (2H, s), 2,70 - 2,77 (2H, m), 2,39 - 2,44 (2H, m) ppm; $^{13}$C-NMR (100 MHz, $CD_3OD$): δ = 176,2 (s), 146,7 (s), 146,2 (s), 145,3 (s), 144,6 (s), 134,8 (s), 133,7 (s), 121,0 (d), 120,6 (d), 118,2 (s), 116,5 (d), 116,4 (d), 108,0 (d), 40,1 (t), 39,2 (t), 32,4 (t) ppm.

**3,4-Dihydroxy-*E*-zimtsäure-(3,4-dihydroxybenzyl)-amid (11)**

Kaffeesäure (5g, 27,8 mmol) wurde in Pyridin (15 ml) gelöst und mit Acetanhydrid (18 ml) bei 100°C 1,5 h gerührt. Die Lösungsmittel wurden abdestilliert und der Rückstand aus Toluol/1,4-Dioxan kristallisiert. Die gereinigte 3,4-Diacetoxy-*E*-zimtsäure (2,67 g, 10,4 mmol) wurde in 1,4-Dioxan (50 ml) gelöst und *N*-Hydroxysuccinimid (1,21 g, 10,5 mmol) sowie *N,N'*-Dicyclohexylcarbodiimid (2,15 g, 10,4 mmol) zugegeben. Nach mehrstündigem Rühren wurde der Niederschlag abfiltriert, das Filtrat eingedampft und mit Methanol kristallisiert. Ausbeute 3,4-Bis(acetyloxy)-*E*-zimtsäure-*N*-succinimidylester: 2,95 g (79 % bez. auf 3,4-Di-O-acetylkaffeesäure); $^1$H-NMR (400 MHz. $CDCl_3$): δ = 7,85 (1H, d, 15,5 Hz), 7,47 (1H, dd, 8,2 Hz, 2 Hz), 7,42 (1H, d, 2 Hz), 7,28 (1H, d, 8,2 Hz), 6,54 (1H, d, 15.5 Hz), 2,88 (4 H, s), 2,13 (3H, 2s), 2,12 (3H, s) ppm.

3,4-Bis(acetyloxy)-*E*-zimtsäure-*N*-succinimidylester (502 mg, 1,39 mmol) und 3,4-Dihydroxybenzylaminhydrobromid (311 mg, 1,41 mmol) wurden unter Stickstoff in Wasser (10 ml) und 1,4-Dioxan (10 ml) gelöst. Nach Zugabe von $NaHCO_3$ (350 mg, 4,16 mmol) wurde die Lösung 2,5 - 3 h bei ca. 70 °C gerührt und abkühlen gelassen. Die Mischung wurde mit Essigsäureethylester extrahiert, die vereinigten organischen Phasen mit Salzsäure (10 %,), Wasser und gesättigter wäßriger NaCl-Lsg. gewaschen, über $Na_2SO_4$ getrocknet, abfiltriert, das Filtrat im Vakuum eingedampft und der Rückstand chromatographisch an Kieselgel gereinigt. Ausbeute: 254 mg (61 %); $^1$H-NMR (400 MHz, $CD_3OD$): δ = 7,41 (1H, d, 15,6 Hz), 7,00 (1H, d, 2,1 Hz), 6,90 (1H, ddd, 8,13 Hz, 2,13 Hz, 0,5 Hz), 6,78 - 6,70 (3H, m), 6,63 (1H, dd, 8,1 Hz, 2,1 Hz), 6,39 (1H, d, 15,6 Hz), 4,32 (2 H, s) ppm; $^{13}$C-NMR (100 MHz, $CD_3OD$): δ = 169,1 (s), 148,8 (s), 146,8 (s), 146,5 (s), 145,7 (s), 142,4 (d), 131,5 (s), 128,4 (s), 122,1 (d), 120,3 (d), 118,4 (d), 116,5 (d), 116,3 (d), 116,0 (d), 115,1 (d), 44,1 (t) ppm; MS (EI): *m/z* = 301,2 (52 %), 179,1 (45 %), 178,2 (26 %), 163,1 (64 %), 138,1 (100 %), 135,1 (22 %), 134,1 (23 %), 123,1 (25 %), 89,1 (34 %), 77,1 (23 %).

In analoger Weise wurden die folgenden Verbindungen in Form farbloser, amorpher Festkörper erhalten:

**3,4-Dihydroxy-*E*-zimtsäure-(4-hydroxy-3-methoxybenzyl)-amid (12):**

$^{1}H$-NMR (400 MHz, $CD_3OD$): δ = 7,42 (1H, d, 15,6 Hz), 7,00 (1H, d, 2,4 Hz), 6,91 - 6,90 (1H, m), 6,90 (1H, ddd, 8Hz, 2,4 Hz, 0,6 Hz), 6,752 (1H, d, 2Hz), 6,750 (1H, d, 8Hz), 6,74 (1H, dd, 8 Hz, 0,6 Hz), 6,39 (1H, d, 15,6 Hz), 4,38 (2H, s), 3,84 (3H, s) ppm; $^{13}C$-NMR (100 MHz, $CD_3OD$): δ =169,1 (s), 149,1 (s), 148,8 (s), 147,0 (s), 146,8 (s), 142,5 (d), 131,5 (s), 128,4 (s), 122,2 (d), 121,6 (d), 118,4 (d), 116,5 (d), 116,2 (d), 115,1 (d), 112,6 (d), 56,4 (q), 44,3 (t) ppm; MS (EI): *m/z* = 315,2 (71 %), 164,1 (21 %), 163,1 (50 %), 153,2 (19 %), 152,1 (100 %), 137,1 (38 %), 136,1 (24 %), 135,1 (15 %), 89,1 (19 %).

**3,4-Dihydroxy-*E*-zimtsäure-(3,5-dimethoxy-4-hydroxybenzyl)-amid (13):**

$^{1}H$-NMR (400 MHz, $CD_3OD$): δ = 7,43 (1H, d, 15,6 Hz), 7,00 (1H, d, 2,1 Hz), 6,91 (1H, ddd, 8,2 Hz, 2,1 Hz, 0,5 Hz), 6,76 (1H, d, 8,2 Hz), 6,62 (2H, s,), 6,40 (1H, d, 15,6 Hz), 4,38 (2H, s), 3,83 (6H, s) ppm, $^{13}C$-NMR (100 MHz, $CD_3OD$): δ = 169,1 (s), 149,4 (s), 148,9 (s), 146,8 (s), 142,6 (d), 135,9 (s), 130,7 (s), 128,3 (2*s), 122,2 (d), 118,3 (d), 116,5 (d), 115,1 (d), 106,2 (2*d), 56,8 (2*q), 44,6 (t) ppm; MS (EI): *m/z* = 345,0 (48 %), 183,1 (19 %), 182,1 (100 %), 179,0 (28 %), 178,0 (16 %), 167,0 (45 %), 163,0 (38 %), 140,0 (15 %), 135,0 (16 %), 89,0 (20 %).

**3,4-Dihydroxy-*E*-zimtsäure-(2,3,4-trihydroxybenzyl)amid (14):**

$^{1}H$-NMR (400 MHz, $CD_3OD$): δ = 7,41 (1H, d, 15 Hz), 6,90 (1H, d, ≈ 2 Hz), 6,89 (1H, dd, 8 Hz, ≈2 Hz), 6,73 (1H, d, 8 Hz), 6,53 (1H, d, 8 Hz), 6,35 (1H, d, 15 Hz), 6,30 (1H, d, 8 Hz), 4,32 ppm (2H, s); MS (ESI pos.) *m/z* = 318,0 (100 %, $[M+H]^+$), 319,1 (17 %, $[M+2H]^+$).

**3,4-Dihydroxyphenylessigsäure-(4-hydroxy-3-methoxybenzyl)-amid (15):**

3,4-Dihydroxyphenylessigsäure (3 g, 17,9 mmol) wurde in Natronlauge (2 mol/l, 26 g) unter Stickstoff gelöst und auf -5 °C gekühlt. Innerhalb von 30 min wurde Chlorameisensäuremethylester (3,73 g, 39,3 mmol) zugegeben. Die Mischung wurde aufwärmen gelassen, noch 30 min bei Raumtemperatur gerührt und mit Salzsäure (5 %) auf pH 3-4 gebracht. Das Produkt wurde mit Essigsäureethylester extrahiert, die organische Phase mit gesättigter NaCl-Lsg. gewaschen, über $Na_2SO_4$ getrocknet, abfiltriert und das Filtrat im Vakuum eingeengt. Ausbeute 3,4-Bis(methoxycarbonyloxy)phenylessigsäure 5,1 g (quant.). $^{1}H$-NMR (400 MHz, $d_6$-DMSO): δ = 7,34 (1H, d, 8 Hz), 7,30 (1H, d, 2 Hz), 7,23 (1H, dd, 8 Hz, 2 Hz), 3,82 (6H, s), 3,61 (2H, s) ppm; MS (ESI pos.): *m/z* = 301,99 (100 %, $[M+NH_4]^+$).

3,4-Bis(methoxycarbonyloxy)phenylessigsäure (5 g, 17,6 mmol) und N-Hydroxysuccinimid (2,02 g, 17,6 mmol) wurden in 1,4-Dioxan unter Stickstoff gelöst. Zu der Lösung wurde N,N-Dicyclohexylcarbodiimid (3,63 g, 17,6 mmol) gegeben und die trübe werdende Mischung 72 h bei Raumtemperatur gerührt. Das Gemisch wird filtriert und das Filtrat im Vakuum eingeengt. Der Rückstand wird an Kieselgel mit dem Eluenten Essigsäureethylester chromatographisch gereinigt. Ausbeute 3,4-Bis(methoxycarbonyloxyphenyl)essigsäure-N-succinimidylester: 5,94 g (89 % d. Th.). $^{1}H$-NMR (400 MHz, $d_6$-DMSO): δ = 7,45-7,40 (2H, m), 7,33 (1H, dd, 8 Hz, 2 Hz), 4,20 (2H, s), 3,84 (6H, s), 2,81 (4H, s) ppm.

3,4-Bis(methoxycarbonyloxy)phenylessigsäure-N-succinimidylester (500 mg, 1,76 mmol) und 4-Hydroxy-3-methoxybenzylaminhydrochlorid wurden in 20 ml 1,4-Dioxan und 20 ml Wasser unter Stickstoff gelöst und Natriumhydrogencarbonat (148 mg, 1,76 mmol) zugegeben. Die Mischung wurde 2 h auf 80°C erwärmt, mit weiterem Natriumhydrogencarbonat versetzt (325 mg, 3,87 mmol), noch 1 h bei 80°C gerührt und abkühlen gelassen. Die orangerote Mischung wurde mit Salzsäure (5 %) angesäuert und mit Essigsäureethylester extrahiert. Die organische Phase wurde mit gesättigter NaCl-Lsg. gewaschen, über $Na_2SO_4$ getrocknet, abfiltriert und das Filtrat im Vakuum eingeengt. Der Rückstand wurde an Kieselgel mit dem Eluenten Essigsäureethylester chromatographisch gereinigt. Ausbeute 3,4-Dihydroxyphenylessigsäure-(4-hydroxy-3-methoxybenzyl)-amid 340 mg (63 % d. Th.).

$^{1}H$-NMR (400 MHz, $d_6$-DMSO): δ = 8,78 (1H, s), 8,76 (1H, s), 8,66 (1H, s), 8,26 (1H, t, 6 Hz), 6,76-6,59 (5H, m), 6,52 (1H, dd, ( Hz, 2Hz), 4,14 (2H, d, 6 Hz), 3,66 (3H, s), 3,24 (2H, s) ppm; MS (ESI pos.) *m/z* = 303,96 (100 %, $[M+H]^+$), 606,6 (30 %, $[2M+H]^+$).

In analoger Weise wurden die folgende Verbindung in Form eines farblosen, amorphen Festkörpers erhalten:

**3,4-Dihydroxyphenylessigsäure- (3,4-dihydroxybenzyl)-amid (16):**

$^{1}H$-NMR (400 MHz, $CDCl_3$): δ = 6,74-6,65 (4H, m), 6,61-6,54 (2H, m), 4,19 (2H, s), 3,36 (2H, s) ppm; MS (ESI neg.): *m/z* = 288,03 (100 %, $[M-H]^-$), 333,58 (17 %, $[M-H+HCOOH]^-$), 576,70 (13 %, $[2M-H]^-$).

## Experimente

### Aktivität als Radikalfänger

Die Aktivität der beispielhaften Verbindungen **1** bis **16** als Radikalfänger wurde mit der herkömmlicher Radikalfänger verglichen. Dabei wurde der DPPH-(1,1-Diphenyl-2-picryl-hydrazyl)-Test zur Beseitigung von Radikalen angewendet.

DPPH wurde in Methanol zu einer Konzentration von 100 μmol/l gelöst. Eine Reihe von Verdünnungen der beispielhaften Verbindungen, Vitamin C, α-Tocopherol, BHT, Ferulasäure sowie des natürlich vorkommenden 4-Hydroxy-3-methoxy-E-zimtsäure-(2-[4-hydroxyphenyl]ethyl)-amids wurden in Methanol hergestellt. Methanol diente als Kontrolle. 2500 μl der DPPH-Lösung wurden mit 500 μl einer jeden Testlösung gemischt und die Abnahme der Absorption bei 515 nm solange abgelesen, bis die Abnahme kleiner 2 % pro Stunde war. Die Aktivität der Testsubstanzen als Radikalfänger wurde nach folgender Gleichung berechnet:

$$\text{Aktiv. als Radikalfänger (\%)} = 100 - \text{(Absorption der Testverbindungen)/(Absorption der Kontrolle)} \times 100.$$

Aus der Aktivität als Radikalfänger (%) in einer Reihe von Verdünnungen von Testverbindungen wurde für jede Testverbindung die effektive relative Konzentration $EC_{50}$ (bezogen auf die anfangs vorhandene Konzentration an DPPH, EC = c (Testverbindung)/c(DPPH)) einer Testverbindung berechnet, bei der das Radikal DPPH um 50 % beseitigt wurde. Die Ergebnisse sind in Tabelle 1 dargestellt:

Tabelle 1

| **Testverbindung** | **$EC_{50}$ / (mol/mol)** |
|---|---|
| Beispiel **1** | 0,1 |
| Beispiel **2** | 0,097 |
| Beispiel **3** | 0,097 |
| Beispiel **4** | 0,1 |
| Beispiel **5** | 0,103 |
| Beispiel **6** | 0,097 |
| Beispiel **7** | 0,094 |
| Beispiel **8** | 0,119 |
| Beispiel **9** | 0,07 |
| Beispiel **10** | 0,069 |
| Beispiel **11** | 0,043 |
| Beispiel **12** | 0,089 |
| Beispiel **13** | 0,1 |
| Beispiel **14** | 0,054 |
| Beispiel **15** | 0,105 |
| Beispiel **16** | 0,053 |
| 4-Hydroxy-3-methoxy-E-zimtsäure-(2-[4-hydroxyphenyl]-ethyl)-amid | 0,441 |
| Vitamin C | 0,270 |
| α-Tocopherol | 0,250 |
| Ferulasäure | 0,350 |
| BHT | 0,240 |

### Aktivität als Antioxidantien

Die Aktivität der beispielhaften Verbindungen **1** bis **16** als Antioxidantien wurde mit der herkömmlicher Antioxidantien verglichen. Als Testsystem wurde die beschleunigte Autoxidation von Lipiden durch Luft mit oder ohne Antioxidans mit Hilfe der Rancimat-Apparatur angewendet (Rancimat ist ein eingetragenes Warenzeichen der Metrohm AG, Herisau, Schweiz).

Die beispielhaften Verbindungen, Vitamin C, α-Tocopherol, BHT, Ferulasäure sowie das natürlich vorkommende 4-Hydroxy-3-methoxy-E-zimtsäure-(2-[4-hydroxy-phenyl]ethyl)-amid wurden in Methanol oder Aceton gelöst und von der jeweiligen Testlösung 100 µl zu einer vorbereiteten Ölprobe (Sojaöl, über Alumina Typ N gereinigt) von 3 g gegeben. In eine Kontrollprobe wurde nur Lösungsmittel gegeben. Durch die auf 100 °C aufgeheizte die Testlösung enthaltende Ölprobe wurde ein konstanter, trockener Luftstrom (20 l/h) geblasen und die flüchtigen Oxidationsprodukte (vorwiegend kurzkettige Fettsäuren wie Ameisen- oder Essigsäure) in einer Vorlage mit Wasser gesammelt. Die Leitfähigkeit dieser wäßrigen Lösung wurde kontinuierlich gemessen und dokumentiert. Die Oxidation von (ungesättigten) Fetten verläuft dabei eine Zeitlang nur sehr langsam und steigt dann plötzlich stark an. Die Zeit bis zum Anstieg wird als Induktionsperiode (IP) bezeichnet.

Nach der folgenden Gleichung wurde der antioxidative Index (AOI) erhalten:

$$AOI = IP_{(\text{mit Testlösung})} / IP_{(\text{Kontrollprobe})}$$

Die Ergebnisse sind in Tabelle 2 dargestellt:

Tabelle 2

| **Testverbindung** | **AOI mit 0,05% Testsubstanz** |
|---|---|
| Beispiel **1** | 11,41 |
| Beispiel **2** | 7,58 |
| Beispiel **3** | 11,7 |
| Beispiel **4** | 17,3 |
| Beispiel **5** | 21,4 |
| Beispiel **6** | 13,9 |
| Beispiel **7** | 7,7 |
| Beispiel **8** | 13,2 |
| Beispiel **9** | 19,8 |
| Beispiel **10** | 24,6 |
| Beispiel **11** | 14,9 |
| Beispiel **12** | 14,3 |
| Beispiel **13** | 3,6 |
| Beispiel **14** | 27,5 |
| Beispiel **15** | 7,84 |
| Beispiel **16** | 8,84 |
| 4-Hydroxy-3 -methoxy-E-zimtsäure-(2-[4-hydroxyphenyl]-ethyl)-amid | 1,41 |
| Vitamin C | 1,17 |
| α-Tocopherol | 5,05 |
| Ferulasäure | 1,79 |
| BHT | 4,77 |

**Patentansprüche**

1. Phenolsäureamide hydroxysubstituierter Benzylamine der allgemeinen Formel (I)

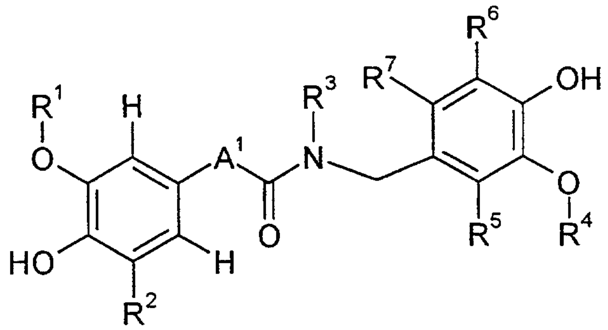

(I)

wobei

$A^1$ eine Gruppe $-CH_2-$, eine Gruppe $-CH_2-CH_2-$ oder eine Gruppe -CH=CH- darstellt, wobei die letztere sowohl in der (Z)- als auch in der (E)-Konfiguration vorliegen kann,

und

$R^1$ ein Wasserstoffatom oder eine Alkylrest mit 1 bis 4 Kohlenstoffatomen darstellt,

und

$R^2$ ein Wasserstoffatom oder eine Gruppe $-O-R^8$ darstellt, in der $R^8$ ein Wasserstoffatom oder ein Alkylrest mit 1 bis 4 Kohlenstoffatomen bedeutet,

und

$R^3$ ein Wasserstoffatom, einen Acyl-, Alkyl- oder Alkenylreste bis 22 Kohlenstoffatome oder eine Gruppe $-(CH_2-CH_2-O-)_nH$ darstellt, wobei n 1 bis 15 sein kann,

und

$R^4$ ein Wasserstoffatom oder eine Alkylrest mit 1 bis 4 Kohlenstoffatomen darstellt, mit der Maßgabe, daß mindestens einer der beiden Reste $R^1$ und $R^4$ ein Wasserstoffatom darstellt,

und

$R^5$, $R^6$ und $R^7$ unabhängig voneinander Wasserstoffatome oder Gruppen $-O-R^9$ darstellen, wobei $R^9$ ein Wasserstoffatom oder ein Alkylrest mit 1 bis 4 Kohlenstoffatomen ist.

2. Phenolsäureamide nach Anspruch 1, **dadurch gekennzeichnet, daß** sie ausgewählt sind aus der Gruppe umfassend

4-Hydroxy-3-methohy-*E*-zimtsäure-(3,4-dihydroxybenzyl)-amid,
3, 5-Dimethoxy-4-hydroxy-*E*-zimtsäure-(3,4-dihydroxybenzyl)-amid,
4-Hydroxy-3-methoxy-*E*-zimtsäure-(2,3,4-trihydroxybenzyl)-amid,
3,5-Dimethoxy-4-hydroxy-*E*-zimtsäure-(2,3,4-trihydroxybenzyl)-amid,
4-Hydroxy-3-methoxy-*E*-zimtsäure-(3,4,5-trihydroxybenzyl)-amid,
4-Hydroxy-3-methoxyphenylessigsäure-(3,4-dihydroxybenzyl)-amid,
3-(4-Hydroxy-3-methoxyphenyl)-propansäure-(3,4-dihydroxybenzyl)-amid,
3-(3,4-Dihydroxyphenyl)-propansäure-(4-hydroxy-3-methoxybenzyl)-amid,
3-(3,4-Dihydroxyphenyl)-propansäure-(3,4-dihydroxybenzyl)-amid,
3-(3,4-Dihydroxyphenyl)-propansäure-(2,3,4-trihydroxybenzyl)-amid,
3,4-Dihydroxy-E-zimtsäure-(3,4-dihydroxybenzyl)-amid,
3,4-Dihydroxy-*E*-zimtsäure-(4-hydroxy-3-methoxybenzyl)-amid,
3,4-Dihydroxy-*E*-zimtsäure-(3,5-dimethoxy-4-hydroxybenzyl)-amid,

3,4-Dihydroxy-*E*-zimtsäure-(2,3,4-trihydroxybenzyl)-amid,
3,4-Dihydroxyphenylessigsäure-(4-hydroxy-3-methoxybenzyl)-amid und
3,4-Dihydroxyphenylessigsäure-(3,4-dihydroxybenzyl)-amid.

**3.** Verfahren zur Herstellung von Phenolsäureamiden hydroxysubstituierter Benzylamine der allgemeinen Formel I

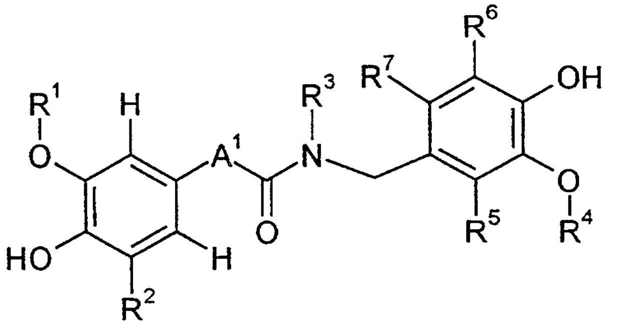

(I)

wobei

$A^1$ eine Gruppe $-CH_2-$, eine Gruppe $-CH_2-CH_2-$ oder eine Gruppe $-CH=CH-$ darstellt, wobei die letztere sowohl in der (Z)- als auch in der (E)-Konfiguration vorliegen kann,

und

$R^1$ ein Wasserstoffatom oder eine Alkylrest mit 1 bis 4 Kohlenstoffatomen darstellt,

und

$R^2$ ein Wasserstoffatom oder eine Gruppe $-O-R^8$ darstellt, in der $R^8$ ein Wasserstoffatom oder ein Alkylrest mit 1 bis 4 Kohlenstoffatomen bedeutet,

und

$R^3$ ein Wasserstoffatom, einen Acyl-, Alkyl- oder Alkenylreste bis 22 Kohlenstoffatome oder eine Gruppe $-(CH_2-CH_2-O-)_nH$ darstellt, wobei n 1 bis 15 sein kann,

und

$R^4$ ein Wasserstoffatom oder eine Alkylrest mit 1 bis 4 Kohlenstoffatomen darstellt, mit der Maßgabe, daß mindestens einer der beiden Reste $R^1$ und $R^4$ ein Wasserstoffatom darstellt,

und

$R^5$, $R^6$ und $R^7$ unabhängig voneinander Wasserstoffatome oder Gruppen $-O-R^9$ darstellen, wobei $R^9$ ein Wasserstoffatom oder ein Alkylrest mit 1 bis 4 Kohlenstoffatomen ist

dergestalt, daß man eine in Form des Säurechlorids, des Säureanhydrids oder eines Säureesters von gegebenenfalls substituierten Phenolen, N-Hydroxysuccinimid oder N-Hydroxybenzotriazol aktivierte, gegebenenfalls an den phenolischen OH-Gruppen geschützte Phenolsäure mit einem gegebenenfalls an den phenolischen OH-Gruppen geschützen Benzylamin oder dessen Ammoniumsalz, gegebenenfalls in Gegenwart von Lösemitteln und Hilfsbasen umsetzt und die gegebenenfalls vorhandenen Schutzgruppen abspaltet.

**4.** Verfahren nach Anspruch 3, **dadurch gekennzeichnet, daß** man als aktivierte Phenolsäureester 4-Hydroxy-3-methoxy-E-zimtsäure-N-succinimidylester, 3,5-Dimethoxy-4-hydroxy-E-zimtsäure-N-succinimidyl-ester, 3,4-Bis(acetyloxy)-E-zimtsäure-N-succinimidyl-ester, 3,4-Bis(methoxycarbonyloxy)-E-zimtsäure-N-succinimidylester, 4-Hydroxy-3-methoxy-phenylessigsäure-N-succinimidylester, 3,4-Bis(methoxycarbonyloxy)-phenylessigsäure-N-succinimidylester, 3-(4-Hydroxy-3-methoxyphenyl)-propansäure-N-succinimidylester und 3-(3,4-Dihydroxyphenyl)-propansäure-N-succinimidylester vewendet.

**5.** Verfahren nach Anspruch 3 und 4, **dadurch gekennzeichnet, daß** man als hydroxysubstituierte Benzylamine 3,4-Dihydroxybenzylamin, 4-Hydroxy-3-methoxybenzylamin, 3,4-Dimethoxy-4-hydroxybenzylamin, 2,3,4-Trihydroxybenzylamin, oder 3,4,5-Trihydroxybenzylamin oder die jeweiligen Ammoniumsalze verwendet.

**6.** Verwendung der Phenolsäureamide nach Anspruch 1 oder 2 zur Verwendung als Antioxidantien oder als Radikalfänger.

**7.** Kosmetische oder pharmazeutische Zusammensetzung umfassend einen wirksamen Gehalt wenigstens eines der Phenolsäureamide gemäß der Ansprüche 1 oder 2.

**8.** Verwendung der Phenolsäureamide nach Anspruch 1 oder 2 zur Verwendung als Antioxidantien oder Radikalfänger in Nahrungsmitteln.

## Claims

**1.** Phenolic acid amides of hydroxy-substituted benzylamines corresponding to the general formula (I)

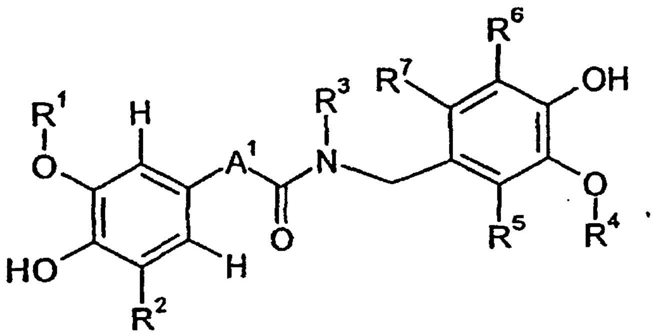

(I)

wherein

$A^1$ represents a $CH_2$ group, a $CH_2$-$CH_2$ group or a CH=CH group, wherein the latter may be present both in the (Z) and in the (E) configuration,

and

$R^1$ represents a hydrogen atom or an alkyl radical having 1 to 4 carbon atoms,

and

$R^2$ represents a hydrogen atom or an O-$R^8$ group in which $R^8$ means a hydrogen atom or an alkyl radical having 1 to 4 carbon atoms,

and

$R^3$ represents a hydrogen atom, an acyl, alkyl or alkenyl radical up to 22 carbon atoms or a $(CH_2\text{-}CH_2\text{-}O\text{-})_nH$ group, wherein n may be 1 to 15,

and

$R^4$ represents a hydrogen atom or an alkyl radical having 1 to 4 carbon atoms, provided that at least one of the two radicals $R^1$ and $R^4$ represents a hydrogen atom,

and

$R^5$, $R^6$ and $R^7$, independently of one another, represent hydrogen atoms or O-$R^9$ groups, wherein $R^9$ is a hydrogen atom or an alkyl radical having 1 to 4 carbon atoms.

2. Phenolic acid amides according to claim 1, **characterised in that** they are selected from the group comprising

4-hydroxy-3-methoxy-*E*-cinnamic acid - (3,4-dihydroxybenzyl) amide
3,5-dimethoxy-4-hydroxy-*E*-cinnamic acid-(3,4-dihydroxybenzyl) amide
4-hydroxy-3-methoxy-*E*-cinnamic acid-(2,3,4-trihydroxybenzyl) amide
3,5-dimethoxy-4-hydroxy-*E*-cinnamic acid-(2,3,4-trihydroxybenzyl) amide
4-hydroxy-3-methoxy-*E*-cinnamic acid - (3,4,5-trihydroxybenzyl) amide
4-hydroxy-3-methoxyphenylacetic acid-(3,4-dihydroxybenzyl) amide
3-(4-hydroxy-3-methoxyphenyl)-propionic acid-(3,4-dihydroxybenzyl) amide
3-(3,4-dihydroxyphenyl)-propionic acid-(4-hydroxy-3-methoxybenzyl) amide
3-(3,4-dihydroxyphenyl)-propionic acid-(3,4-dihydroxybenzyl) amide
3-(3,4-dihydroxyphenyl)-propionic acid-(2,3,4-trihydroxybenzyl) amide
3,4-dihydroxy-*E*-cinnamic acid-(3,4-dihydroxybenzyl) amide
3,4-dihydroxy-*E*-cinnamic acid-(4-hydroxy-3-methoxybenzyl) amide
3,4-dihydroxy-*E*-cinnamic acid-(3,5-dimethoxy-4-hydroxybenzyl) amide
3,4-dihydroxy-*E*-cinnamic acid-(2,3,4-trihydroxybenzyl) amide
3,4-dihydroxyphenylacetic acid-(4-hydroxy-3-methoxybenzyl) amide and
3,4-dihydroxyphenylacetic acid-(3,4-dihydroxybenzyl) amide.

3. A process for the preparation of phenolic acid amides of hydroxy-substituted benzylamines corresponding to the general formula I

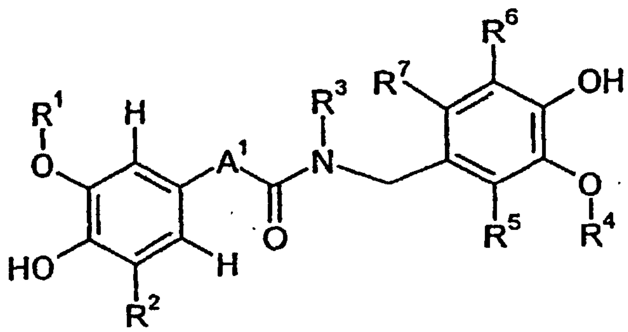

(I)

wherein

$A^1$ represents a $CH_2$ group, a $CH_2$-$CH_2$ group or a CH=CH group, wherein the latter may be present both in the (Z) and in the (E) configuration,

and

$R^1$ represents a hydrogen atom or an alkyl radical having 1 to 4 carbon atoms,

and

$R^2$ represents a hydrogen atom or an O-$R^8$ group in which $R^8$ means a hydrogen atom or an alkyl radical having 1 to 4 carbon atoms,

and

$R^3$ represents a hydrogen atom, an acyl, alkyl or alkenyl radical up to 22 carbon atoms or a $(CH_2$-$CH_2$-$O$-$)_nH$ group, wherein n may be 1 to 15,

and

$R^4$ represents a hydrogen atom or an alkyl radical having 1 to 4 carbon atoms, provided that at least one of the two radicals $R^1$ and $R^4$ represents a hydrogen atom,

and

$R^5$, $R^6$ and $R^7$, independently of one another, represent hydrogen atoms or O-$R^9$ groups, wherein $R^9$ is a hydrogen atom or an alkyl radical having 1 to 4 carbon atoms,

in such a manner that an activated phenolic acid in the form of the acid chloride, the acid anhydride or an acid ester of optionally substituted phenols, N-hydroxysuccinimide or N-hydroxybenzotriazole and optionally protected on the phenolic OH groups is reacted with a benzylamine optionally protected on the phenolic OH groups or with the ammonium salt thereof, optionally in the presence of solvents and auxiliary bases, and the optionally present protective groups are cleaved.

4. A process according to claim 3, **characterised in that** activated phenolic acid esters used are 4-hydroxy-3-methoxy-E-cinnamic acid-N-succinimidyl ester, 3,5-dimethoxy-4-hydroxy-E-cinnamic acid-N-succinimidyl ester, 3,4-bis (acetyloxy)-E-cinnamic acid-N-succinimidyl ester, 3,4-bis(methoxycarbonyloxy)-E-cinnamic acid-N-succinimidyl ester, 4-hydroxy-3-methoxy-phenylacetic acid-N-succinimidyl ester, 3,4-bis(methoxycarbonyloxy)-phenylacetic acid-N-succinimidyl ester, 3-(4-hydroxy-3-methoxyphenyl)-propionic acid-N-succinimidyl ester and 3-(3,4-dihydroxyphenyl)-propionic acid-N-succinimidyl ester.

5. A process according to claim 3 and 4, **characterised in that** hydroxy-substituted benzylamines used are 3,4-dihydroxybenzylamine, 4-hydroxy-3-methoxybenzylamine, 3,4-dimethoxy-4-hydroxybenzylamine, 2,3,4-trihydroxybenzylamine or 3,4,5-trihydroxybenzylamine or the ammonium salts in each case.

6. The use of the phenolic acid amides according to claim 1 or 2 for use as antioxidants or as radical scavengers.

7. A cosmetic or pharmaceutical composition comprising an effective content of at least one of the phenolic acid amides according to claims 1 or 2.

8. The use of the phenolic acid amides according to claim 1 or 2 for use as antioxidants or radical scavengers in foodstuffs.

**Revendications**

1. Amides d'acides phénoliques et de benzylamines hydroxysubstituées de formule générale I

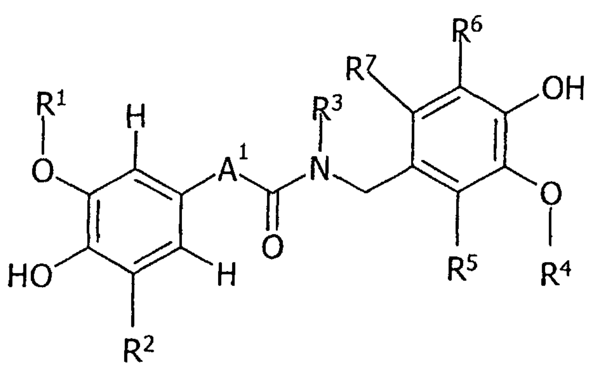

(I)

où

$A^1$ représente un groupe $-CH_2-$, un groupe $-CH_2-CH_2-$ ou un groupe -CH=CH-, ce dernier pouvant être présent aussi bien dans la configuration (Z) que dans la configuration (E),

et

$R^1$ représente un atome d'hydrogène ou un reste alkyle ayant 1 à 4 atomes de carbone,

et

$R^2$ représente un atome d'hydrogène ou un groupe -O-$R^8$ où $R^8$ représente un atome d'hydrogène ou un reste alkyle ayant 1 à 4 atomes de carbone,

et

$R^3$ représente un atome d'hydrogène, un reste acyle, alkyle ou alkylène ayant jusqu'à 22 atomes de carbone ou un groupe $-(CH_2-CH_2-O-)_nH$, où n peut être 1 à 15,

et

$R^4$ représente un atome d'hydrogène ou un reste alkyle ayant 1 à 4 atomes de carbone, à condition qu'au moins l'un des deux restes $R^1$ et $R^4$ représente un atome d'hydrogène,

et

$R^5$, $R^6$ et $R^7$ représentent indépendamment les uns des autres des atomes d'hydrogène ou des groupes -O-$R^9$ où $R^9$ représente un atome d'hydrogène ou un reste alkyle ayant 1 à 4 atomes de carbone.

**2.** Amides d'acides phénoliques selon la revendication 1, **caractérisés en ce qu'**ils sont choisis dans le groupe comprenant

(3,4-dihydroxybenzyl)amide d'acide 4-hydroxy-3-méthoxy-*E*-cinnamique,
(3,4-dihydroxybenzyl)amide d'acide 3,5-diméthoxy-4-hydroxy-*E*-cinnamique,
(2,3,4-trihydroxybenzyl)amide d'acide 4-hydroxy-3-méthoxy-*E*-cinnamique,
(2,3,4-trihydroxybenzyl)amide d'acide 3,5-diméthoxy-4-hydroxy-*E*-cinnamique,
(3,4,5-trihydroxybenzyi)amide d'acide 4-hydroxy-3-méthoxy-*E*-cinnamique,
(3,4-dihydroxybenzyl)amide d'acide 4-hydroxy-3-méthoxyphénylacétique,
(3,4-dihydroxybenzyl)amide d'acide 3-(4-hydroxy-3-méthoxyphényl)-propanoïque,
(4-hydroxy-3-méthoxybenzyl)amide d'acide 3-(3,4-dihydroxyphényl)-propanoïque,
(3,4-dihydroxybenzyl)amide d'acide 3-(3,4-dihydroxyphényl)propanoïque,
(2,3,4-trihydroxybenzyi)amide d'acide 3-(3,4-dihydroxyphényl)-propanoïque,
(3,4-dihydroxybenzyl)amide d'acide 3,4-dihydroxy-*E*-cinnamique,
(4-hydroxy-3-méthoxybenzyl)amide d'acide 3,4-dihydroxy-*E*-cinnamique,
(3,5-diméthoxy-4-hydroxybenzyl)amide d'acide 3,4-dihydroxy-*E*-cinnamique,
(2,3,4-trihydroxybenzyl)amide d'acide 3,4-dihydroxy-*E*-cinnamique,
(4-hydroxy-3-méthoxybenzyi)amide d'acide 3,4-dihydroxyphénylacétique,
et (3,4-dihydroxybenzyl)amide d'acide 3,4-dihydroxyphénylacétique.

**3.** Procédé de préparation d'amides d'acides phénoliques et de benzylamines hydroxysubstituées de formule générale (I)

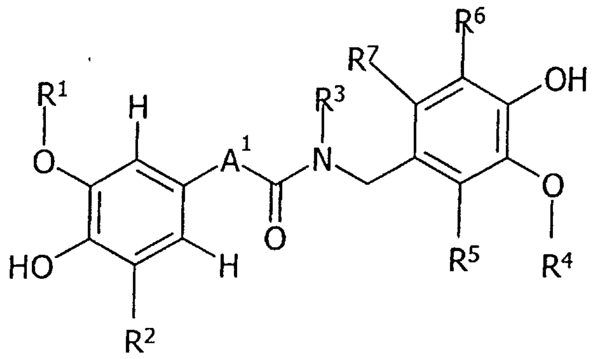

(I)

où

$A^1$ représente un groupe $-CH_2-$, un groupe $-CH_2-CH_2-$ ou un groupe -CH=CH-, ce dernier pouvant être présent aussi bien dans la configuration (Z) que dans la configuration (E),

et

$R^1$ représente un atome d'hydrogène ou un reste alkyle ayant 1 à 4 atomes de carbone,

et

$R^2$ représente un atome d'hydrogène ou un groupe $-O-R^8$ où $R^8$ représente un atome d'hydrogène ou un reste alkyle ayant 1 à 4 atomes de carbone,

et

$R^3$ représente un atome d'hydrogène, un reste acyle, alkyle ou alcényle ayant jusqu'à 22 atomes de carbone ou un groupe $-(CH_2-CH_2-O-)_nH$, où n peut être 1 à 15,

et

$R^4$ représente un atome d'hydrogène ou un reste alkyle ayant 1 à 4 atomes de carbone, à condition qu'au moins l'un des deux restes $R^1$ et $R^4$ représente un atome d'hydrogène,

et

$R^5$, $R^6$ et $R^7$ représentent indépendamment les uns des autres des atomes d'hydrogène ou des groupes $-O-R^9$ où $R^9$ représente un atome d'hydrogène ou un reste alkyle ayant 1 à 4 atomes de carbone,

selon lequel on fait réagir un acide phénolique sous forme du chlorure d'acide, de l'anhydride d'acide ou d'un ester de phénols éventuellement substitués, de N-hydroxysuccinimide ou de N-hydroxybenzotriazole activé, éventuellement protégé au niveau des groupes OH phénoliques avec une benzylamine de même éventuellement protégée au niveau des groupes OH phénoliques ou son sel d'ammonium, éventuellement en présence de solvants et de bases auxiliaires, et on clive les groupes protecteurs éventuellement présents.

**4.** Procédé selon la revendication 3, **caractérisé en ce que** l'on utilise comme esters d'acides phénoliques activés l'ester d'acide 4-hydroxy-3-méthoxy-*E*-cinnamique et de N-succinimide, l'ester d'acide 3,5-diméthoxy-4-hydroxy-E-cinnamique et de N-succinimide, l'ester d'acide 3,4-bis(acétyloxy)-E-cinnamique et de N-succinimide, l'ester d'acide 3,4-bis(méthoxycarbonyloxy)-E-cinnamique et de N-succinimide, l'ester d'acide 4-hydroxy-3-méthoxyphénylacétique et de N-succinimide, l'ester d'acide 3,4-bis(méthoxycarbonyloxy)-phénylacétique et de N-succinimide, l'ester d'acide 3-(4-hydroxy-3-méthoxyphényl)propanoïque et de N-succinimide et l'ester d'acide 3-(3,4-dihydroxyphényl)propanoïque et de N-succinimide.

**5.** Procédé selon les revendications 3 et 4, **caractérisé en ce que** l'on utilise comme benzylamines hydroxysubstituées la 3,4-dihydroxybenzylamine, la 4-hydroxy-3-méthoxybenzylamine, la 3,4-diméthoxy-4-hydroxybenzylamine, la 2,3,4-trihydroxybenzylamine ou la 3,4,5-trihydroxybenzylamine ou les sels d'ammonium correspondants.

**6.** Utilisation des amides d'acides phénoliques selon la revendication 1 ou 2 comme antioxydants ou pièges à radicaux.

**7.** Composition cosmétique ou pharmaceutique contenant une teneur efficace en au moins l'un des amides d'acides phénoliques selon la revendication 1 ou 2.

**8.** Utilisation des amides d'acides phénoliques selon la revendication 1 ou 2 comme antioxydants ou pièges à radicaux dans des aliments.